# EUROPEAN PATENT SPECIFICATION

(11) **EP 2 900 314 B1**
(45) Date of publication and mention of the grant of the patent: **28.02.2018**
(21) Application number: 13842322.3
(22) Date of filing: 23.09.2013
(51) Int. Cl.: A61N 1/04, A61N 1/18, A61N 1/32, A61B 18/14, A61B 18/00

(54) **DEVICES AND METHODS FOR STIMULATION OF HAIR GROWTH**
VORRICHTUNGEN UND VERFAHREN ZUR STIMULIERUNG DES HAARWACHSTUMS
DISPOSITIFS ET PROCÉDÉS DE STIMULATION DE LA POUSSE DES CHEVEUX

(30) Priority: 25.09.2012 US 201213625886
(43) Date of publication of application: 05.08.2015
(73) Proprietor: Innogen Technologies Ltd., 6971005 Tel Aviv (IL)
(72) Inventor: Vernes Rashkovsky, Ines, Savyon (IL); Vaynberg, Boris, Zichron Yaakov (IL)
(74) Representative: Lecomte & Partners
(86) International application number: PCT/IB2013/058763
(87) International publication number: WO 2014/049510

(56) References cited:
- WO-A1-95/09671
- WO-A2-2005/060354
- JP-A- 2005 006 981
- KR-U- 20090 012 226
- KR-U- 20090 012 226
- US-A1- 2003 220 635
- US-A1- 2008 249 350
- US-B1- 6 834 206
- US-B1- 6 834 206

## Description

### FIELD AND BACKGROUND OF THE INVENTION

The invention, in some embodiments, relates to the field of cosmetic treatment, and more particularly, but not exclusively, to devices for stimulation of hair growth.

Hair loss is very common in both men and women. In males, male pattern hair loss, may start at any age after puberty and its frequency and severity increase with advancing age.

During their teens, almost all Caucasian males develop recession of the frontal temporal hairline. Deeper recessions and vertex balding have a later onset in most males. At the age of 70, about 80% of men have more pronounced baldness. In females, female pattern hair loss is less common than male balding but as in males, may start at any age after puberty with an increase in frequency and severity with aging. At the age of 30, around 2-5% of females will have remarkable thinning of hair, rising to 40% at the age of 70. Both male and female pattern hair loss impair the quality of life and cause psychological distress that is more pronounced in females. Moreover with advancing age there is a decrease in the quantity of hair follicles, decrease in hair diameter, a slower growth rate and a higher quantity of hair follicles in the telogen growth phase in both sexes.

Different treatment modalities are currently available for the treatment of male or female pattern hair loss, none of which give satisfactory results:

### (1) Medication

For both male and female pattern hair loss, long-term use of topical minoxidil 2-5% solution can give variable results with the recurrence of hair loss shortly after stopping the medication. Minoxidil is associated with a number of side-effects, including scalp irritation or allergic contact dermatitis. Sometimes, unwanted hair growth elsewhere on the body may be seen, especially with use of the 5% solution.

Oral finasteride is a competitive 5-α-reductase inhibitor, which reduces dihydrotestosterone levels in the scalp and in serum by 70%. This medication can be used in males and can attenuate male pattern hair loss when used over a number of years. A gradual relapse occurs after discontinuation of treatment. Side effects include sexual dysfunction (in 4.2-8.7% of users) and a probable increased risk of prostate cancer.

Oral anti-androgens have been suggested for use in females but proof of their efficacy is still lacking.

### (2) Transplantation

In both males and females, hair transplantation surgery can be performed with variable results. Such procedures are invasive and expensive.

### (3) Light-based devices

A number of products using low level laser therapy (LLLT) for simulation of hair growth have been recently introduced and one of them (HairMax LaserComb®) has obtained FDA approval. The mechanism of action is not known and the efficacy of these LLLT products is very limited.

Document published KR 2009 0012226 U shows a high frequency therapeutic equipment with two separate electrode units.

US patent published US 6,834,206 B1 shows a device for applying a therapeutic signal to a body portion to encourage hair growth and include a first electrode and second electrode.

International patent application published WO 2005/060354 A2 discloses a system for modifying or destroying a tissue. The electrodes surround the tissue.

International patent application published WO 95/09671 A1 discloses an apparatus for promoting hair growth. The exterior of a hood liner carries a plurality of electrodes. When the liner is inserted within the hood, and the hood is placed over a subject's head, the electrodes are positioned closely proximate to the subject's scalp, but do not touch the skin.

### SUMMARY OF THE INVENTION

The present invention relates to a device for stimulation of hair re-growth on an area of skin of a subject in accordance with appended independent claim 1 and appended dependent claims 2-12.

The present invention relates to devices comprising use of localized electrical energy to stimulate an area of hair loss, thereby promoting re-growth of hair in the area. In some embodiments, the area of hair loss is an area of the scalp. In some embodiments, a controlled wound is induced, wherein healing of the wound induces hair re-growth. In some embodiments, the device of the present invention is safe and simple to use, typically producing predictable and reproducible results, typically independent of skin type, in preferred embodiments with little or no pain.

According to some embodiments, there is provided a device for stimulation of hair re-growth on an area of skin of a subject, the device comprising an array of electrodes comprising at least one contact electrode, and at least one ground electrode, each of the electrodes configured to conduct electrical current to a surface of an area of skin via a contact surface of an electrode, wherein the electrical current delivers energy sufficient to stimulate hair growth, e.g.to cause localized heating of the area of skin, wherein the localized heating promotes re-growth of hair in the area. A localized wound is induced, wherein healing of the localized wound stimulates hair re-growth. In some embodiments, the array of electrodes comprises at least two contact electrodes and at least one ground electrode.

According to some embodiments, the device may be configured such that the function of at least one of the electrodes of the array of electrodes can be optionally switched between being a ground electrode and being a contact electrode, for example using a standard switch mechanism as known in the art.

According to some embodiments, the device is configured such that at least one pair of a first electrode and a second electrode of the array of electrodes can be switched between two states: a first state wherein the first electrode is a ground electrode and the second electrode is a contact electrode; and a second state wherein the first electrode is a contact electrode and the second electrode is a ground electrode.

According to some embodiments, the device further comprises a power supply configured to supply a voltage causing the electrical current. In some embodiments, the power supply is configured to supply a voltage causing an electrical current having a frequency in radiofrequency range, optionally in the range of from about 0.2 MHz to about 40 MHz. In some embodiments, the power supply is configured so that the electrical current provides energy in the range of from about 1 to about 200 mJ through each contact surface of each contact electrode.

According to some embodiments, the contact surface of each of contact electrodes has a cross-sectional dimension in the range of from about 25 to about 500 µm. The device is configured to identify a measure of quality of the electrical contact between electrode surfaces and skin surface at a given moment. In some embodiments, the device is configured to adjust parameters of an electric current through the contact surface dependent on the measure of contact quality.

According to some embodiments, the device is configured to measure the impedance of a circuit comprising the contact electrode, the ground electrode, the power supply and the skin. In some embodiments, the device is configured to adjust parameters of an electric current through the contact surfaces depending on the measured impedance.

According to some embodiments, the device comprises a plurality of protruding elements extending from a base section, wherein the contact electrodes are embedded within the protruding elements.

According to some embodiments, the protruding elements comprise substantially parallel, elongated elements.

In some embodiments, in a single protruding element are embedded a contact electrode and a ground electrode. In some embodiments, in a single protruding element are embedded at least two ground electrodes and a contact electrode. The ground electrodes are embedded within the protruding elements.

According to some embodiments, a total contact surface area of contact electrodes is substantially equal to a total surface area of ground electrodes.

According to some embodiments, a total contact surface area of contact electrodes is equal to or less than a total surface area of ground electrodes.

According to some embodiments, the device comprises two parts, a first part comprising a lead connected to the power supply and a second part comprising the contact surfaces of contact electrodes, wherein the first part and the second part are reversibly couplable, wherein in at least one coupled state, there is electrical communication between the lead of the first part and the contact surfaces of the second part. In some embodiments, the second part comprising the contact surfaces is disposable.

According to some embodiments, the electrodes comprise a two-dimensional array. There is provided a method for stimulation of hair re-growth, the method comprising providing a device for stimulation of hair re-growth on an area of skin of a subject, the device comprising an array of at least one, optionally at least two contact electrodes, configured to conduct electrical current to a surface of an area of skin via a contact surface; contacting the device with the area of skin; and passing an electrical current through the contact surface of the contact electrode, wherein the electrical current delivers energy sufficient to cause a localized heating effect in the area of skin, thereby promoting hair re-growth. A localized wound is induced, wherein healing of the wound stimulates hair-regrowth. The device is moved across the area of skin at a speed in the range of from about 0.5 to about 20 cm/sec. The device comprises an array of at least two contact electrodes. A device used in the method for stimulation of hair growth may comprise any of the devices described herein. The device is configured to conduct electrical current only when in stationary contact with an area of skin (*i.e* stamping mode).. The device is configured to intermittently conduct electrical current while moving across an area of skin.

Unless otherwise defined, all technical and scientific terms used herein have the same meaning as commonly understood by one of ordinary skill in the art to which the invention pertains. In case of conflict, the specification, including definitions, will control.

As used herein, the terms "comprising", "including", "having" and grammatical variants thereof are to be taken as specifying the stated features, integers, steps or components but do not preclude the addition of one or more additional features, integers, steps, components or groups thereof. These terms encompass the terms "consisting of' and "consisting essentially of'.

As used herein, the indefinite articles "a" and "an" mean "at least one" or "one or more" unless the context clearly dictates otherwise.

### BRIEF DESCRIPTION OF THE FIGURES

Some embodiments of the invention are described herein with reference to the accompanying figures. The description, together with the figures, makes apparent to a person having ordinary skill in the art how some embodiments of the invention may be practiced. The figures are for the purpose of illustrative discussion and no attempt is made to show structural details of an embodiment in more detail than is necessary for a fundamental understanding of the invention. For the sake of clarity, some objects depicted in the figures are not to scale.

In the Figures:
FIG. 1A is a schematic side view of a device for stimulating hair growth;
FIG. 1B is a lower cross-sectional view of the device of Fig. 1A showing a contact electrode embedded in each of the teeth of the device;
FIG. 2A is a schematic side view of a device for stimulating hair growth;
FIG. 2B is a lower cross-sectional view of the device of Fig. 2A showing a contact electrode embedded in each of the teeth of the device;
FIG. 3A is a schematic side view of an additional alternative embodiment of the device of the present invention;
FIG. 3B is a lower cross-sectional view of an embodiment of the device of Fig. 3A, showing a contact electrode and a ground electrode of different size embedded in the teeth of the device;
FIG. 3C is a lower cross-sectional view of an embodiment of the device of Fig. 3A, showing a contact electrode and a ground electrode of equal size embedded in the teeth of the device;
FIG. 3D is a lower cross-sectional view of an embodiment of the device of Fig. 3A, showing one contact electrode and two ground electrodes embedded in the teeth of the device;
FIG. 4A is a schematic side view of an additional device for stimulating hair growth;
FIG. 4B is a lower cross-sectional view of the device of Fig. 4A showing a contact electrode embedded in each of the teeth of the device;
FIG. 4C is a lower cross-sectional view of the device of Fig. 4A showing alternate contact electrodes and ground electrodes embedded in adjacent teeth of the device;
FIGS. 5A and 5B show schematic side views of an additional alternative device for stimulating hair growth;
FIG. 5C shows a front view of the device of Fig. 5B;
FIG. 6 shows a perspective front view of an additional alternative device for stimulating hair growth;
FIGS. 7A, 7B and 7C show schematic representations of the device of Fig. 2a, comprising an array of 5 groups of electrodes; and
FIGS. 8A and 8B are photographs of a patient before and after treatment, respectively.

### DESCRIPTION OF SOME EMBODIMENTS OF THE INVENTION

The invention, in some embodiments, relates to the field of cosmetic treatment, and more particularly, to devices for stimulation of hair growth. The principles, uses and implementations of the teachings herein may be better understood with reference to the accompanying description and figures. Upon perusal of the description and figures present herein, one skilled in the art is able to implement the invention without undue effort or experimentation. In the figures, like reference numerals refer to like parts throughout. There is provided a method for the cosmetic treatment of hair loss. The method comprises providing a device for stimulation of hair re-growth on an area of skin of a subject, the device comprising an array of electrodes, each of the electrodes configured to conduct electrical current to a surface of an area of skin which the electrode contacts wherein the electrical current delivers electrical energy sufficient to cause a localized thermal effect at the area of skin; and while moving the device relative to the skin surface intermittently conducting such electrical current to the skin through the electrodes to cause a plurality of localized thermal zones.

As used herein, the term "thermal zone" refers to a zone (volume) of tissue in an area of skin located directly below a contact electrode where upon application of electrical current according to the teachings herein, the tissue temperature is increased to a desired level. In some embodiments a desired level is about 100°C to cause ablation. A desired level is in the range 43-100 °C to cause coagulation. A desired level is below about 43 °C to cause heating without substantial irreversible effects. Ablation and coagulation may be associated with wound creation and wound healing processes. Heating below 43 °C does not introduce wounding but stimulates hair regrowth nevertheless. The level of heating effect is controlled by the level of RF energy delivered to the tissue. The localized thermal effect causes at least one localized wound, wherein healing of the localized wound stimulates hair growth.

The parameters of the electric current (e.g., power, frequency, voltage) may be varied so as to maintain a substantially similar level of energy delivered to an area independent of the total surface area of contact surfaces contacting the skin surfaces. As used herein, the term "surface area" refers to an area ordinarily contacted when properly used by a person in accordance with the instructions.

The method described herein may be implemented using any suitable device. It is preferred to use a dermatological treatment device as described herein.

According to some aspects of the present invention, there is provided a device for stimulation of hair re-growth on an area of skin of a subject, the device comprising an array of contact electrodes, each contact electrode being configured to conduct electrical current to a surface of an area of skin via a contact surface. The electrical current delivers electrical energy sufficient to cause a localized thermal effect at the area of skin, thereby stimulating hair re-growth. In some embodiments, the localized thermal effect causes at least one localized wound, wherein healing of the wound stimulates hair re-growth.

The devices of the present invention are applied to the outer layer (epidermis) of the skin, which is less conductive than underlying layers, such that the density of electrical current is relatively high in the epidermis. The outer layer of the epidermis, the stratum corneum, is dielectric and resistant to electrical breakdown up to a certain voltage level. When the voltage exceeds the electrical breakdown threshold, an electrical discharge takes place, which affects the stratum corneum. The electrical discharge causes shallow wounds.

Preferably, a plurality of small wounds are produced and allowed to heal in an area of skin, rather than damaging a large contiguous area of skin, a technique known as fractional technology. It is believed that the body can more easily heal a small damaged area surrounded by healthy tissue then a large damaged area. The size and depth of the wounds is determined by the size of the electrodes applied to the skin, as well as the specific parameters of the electrical current, such as energy, power, voltage and frequency.

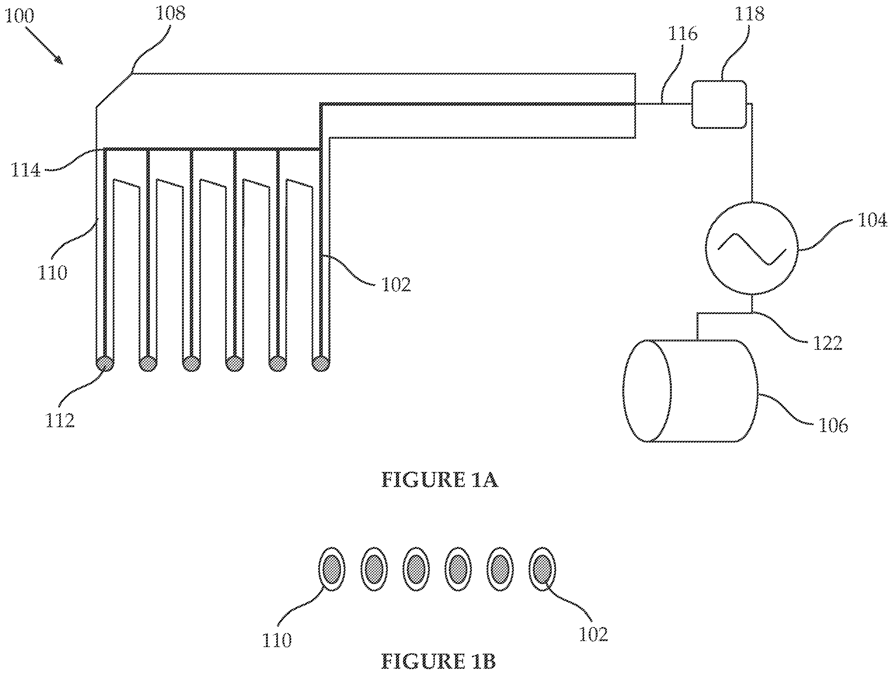

An exemplary device **100,** is shown in Fig. 1A.

As seen in Fig. 1A, device **100** comprises an array of contact electrodes **102,** connected via an alternating current power supply **104** to at least one ground electrode **106,** wherein power supply **104** is configured to supply alternating electric current, typically at radiofrequency voltage. Device **100** further comprises a housing **108,** having a plurality of protruding elements comprising elongated teeth **110,** such that housing **108** resembles, and can function as, a comb. A portion of housing **108** enclosing contact electrodes **102** is fashioned from an insulating material, such as polycarbonate, thereby enabling a user to directly hold housing **108** in the hand during use, while exposing ground electrode **106** to the skin of the user, such that when a user holds device **100,** the hand of the user makes electrical contact with ground electrode **106.**

Housing **108** is of suitable dimensions to enable device **100** to be comfortably held in the hand of a user during operation. Housing **108** may optionally further be provided with a gripping area or handle (not shown).

Each of contact electrodes **102** is embedded within a distal end of one of elongated, protruding teeth **110,** such that a contact surface **112** of each of contact electrodes **102** is exposed. One or more conductors **114,** providing electrical contact between power supply**104** through one or more contact electrode leads **116** and contact electrodes **102,** is insulatingly embedded within a proximal portion of housing **108.**

Contact surfaces **112** are preferably continuously curved, such as, for example, dome-shaped.

Curved surfaces, such as dome shapes, allow reasonable electrical contact with the flexible skin surface without requiring excessive pressure and allow dragging or continuous or stepwise movement across a skin surface (during a combing motion, see below) without causing pain or discomfort.

Elongated teeth **110** are preferably capable of being easily bent without breaking, such that any existing hairs in the treated area are easily separated to enable contact surface **112** to contact the skin surface. The profile of each of elongated teeth **110** of device may be of any suitable shape, such as, for example, circular or elliptical (as in Fig. 1B). Alternatively, elongated teeth **110** may be any suitable cross-sectional shape, preferably of rigidity, dimensions and material similar to those of the teeth of a comb for combing hair.

The cross-sectional dimension of contact surface **112,** such as the diameter of a contact surface **112** of circular or elliptical teeth **110,** influences the size of the thermal zone , such as the size of a wound, induced by each contact surface **112** as well as the parameters of the electrical current required to produce the desired effect. Preferably, the cross-sectional dimension of contact surfaces **112** is in the range of from about 25 to about 500 micrometers, such that the size of a wound is no greater than about 500 micrometers, preferably no greater than about 200 micrometers. Teeth **110** may be flexible, to provide adequate contact of contact surfaces **112** of contact electrodes **102** with a curved skin surface, such as that of the scalp.

The plurality of teeth **110** is any suitable number of teeth **110** greater than 1, preferably greater than 2. Generally, when the number of teeth **110** is small, providing a narrower array, more parallel strips are needed to treat an area of skin and with a larger number of teeth, (wider array) the device is less maneuverable. Hence, the number of teeth **110** should be sufficient to provide fast and efficient treatment of a skin area without the need for an excessive number of repeated applications, but small enough such that the array is of a width which may be contained within housing **108** of a convenient size to be held easily in the hand of the user. In some embodiments, a center-to-center separation distance for adjacent electrodes is in the range of from about 3 mm to about 4 mm. Contact electrodes **102** are arranged as a one-dimensional array (a line), that in some embodiments is oriented substantially perpendicular to the direction in which the device is moved along the skin surface.

In some embodiments, contact electrodes **102** are arranged as a two-dimensional array, in a manner of a comb or brush.

Two-dimensional arrays may comprise an evenly or randomly spaced matrix, of for example 8x8, 12x12, 16x16, 16x24 electrodes, or any other number and configuration of electrodes.

The distance between any two adjacent contact electrodes **102** is any suitable distance which is generally determined according to clinical factors known in the art of fractional technology, and is sufficient to provide effective hair separation. The center-to-center separation distance for adjacent contact electrodes **102** is in the range of from about 1 to about 3 mm. The alternating voltage causing the electrical current supplied by power supply **104** is in the radio frequency (RF) range, preferably in the range of from about 0.2 MHz to about 40 MHz, and more preferably in the range of from about 1 MHz to about 15 MHz. The voltage of alternating current is any suitable voltage that is determined by a person having ordinary skill in the art based on factors such as the cross sectional area of the contact electrodes, the extent of wounding desired and safety factors. That said, in some embodiments, the preferred voltage is between 10 V and 400 V, more preferably between 10 V and 150 V. The current power is preferably such that a desired degree of ablation, coagulation or heating of the fraction of the skin occurs. A pulse width of electrical current is selected so as to be short enough to produce small, spaced-apart wounds as device **100** is moved over the skin surface. According to some embodiments, a pulse width of electrical current is in the range of from about 200 µsec to about 500 msec, preferably from about 1 to about 20 msec,. All contact electrodes **102** are configured to conduct current simultaneously. Individual or subgroups of contact electrodes **102** are activated sequentially, in fixed or random sequences, for example, in order to prevent or minimize pain experienced by the user. The ratio of the volume of heated to non-heated skin within a treated area is low in order to minimize pain and provide faster and more effective healing. Multiple applications of the device may be required in order to produce the desired effect.

Device **100** further comprises a trigger **118** configured to allow the passage of current through a circuit comprising contact electrodes **102,** ground electrodes **106,** power supply **104** and a treated skin surface. Trigger **118** of device **100** is an intermittent trigger, which is automatically activated at predetermined intervals of time, e.g., at a rate of 3 to 10 Hz. Trigger **118** is functionally associated with a timer (not shown).

Alternatively, trigger **118** may be activated manually by the user, or may be activated automatically at predetermined distance intervals as device **100** travels over the skin surface. A trigger is typically an assembly of various electric components, including a microprocessor or printed circuit board, functional to trigger the device to conduct an electric current as described herein. Trigger **118** is functionally associated with a distance-measurer (not shown), the distance measurer configured to determine a distance traveled by the device along a skin surface in a prescribed direction when in contact with the skin surface, allowing the electrical current that causes wounds to be supplied at intervals so that the wounds are separated by a desired distance, e.g., between 1 mm and 5 mm. Any suitable distance measurer may be used in implementing the teachings herein, such as, for example, a mechanical rolling component and/or timer component and/or electrooptical component such as used in a computer mouse.

Device **100** is configured to be hand-operable, allowing a user to manually move device **100** along the surface of the skin in a prescribed direction, in a combing motion, analogous to a conventional hair comb. The device is either held by gripping housing 108, or by holding a handle optionally attached to the device (not shown). Current is periodically applied to the skin during motion of the device over the surface of the skin, under control of trigger **118.** To treat a large surface area, the device is moved in a prescribed direction to treat a first strip of skin, and then relocated and moved in substantially the same way to treat a following strip of skin parallel to the first strip of skin. The device is preferably moved manually along the skin surface by a user, thus avoiding the need for complex control systems.

Device **100** may optionally be configured to conduct electrical current only when in stationary contact with an area of skin, or to conduct electrical current intermittently while moving across an area of skin.

A challenge in implementing the teachings herein relates to safety. Generally, a device is configured to conduct an electric current that is sufficient to cause a desired level of heating effect to the skin area. If the electric current is such that the localized heating is insufficient, the use of the device is likely ineffective. If the electric current is such that heating is too strong, the use of the device is potentially painful or even damaging to the skin. As long as all contact surfaces are in contact with a skin surface, and therefore the total area of contact surfaces in contact with a skin surface is known, a certain predetermined current with specific parameters can be conducted to cause the desired level of heating. However, if not all the contact surfaces are in contact with the skin surface, the total area of contact surfaces in contact with the skin surface is relatively small; if the certain predetermined current is conducted through the relatively small total area, the damage caused at the contacting surfaces may be too strong.

Accordingly, the device is configured to identify a measure of the total surface area of contact surfaces (typically, the number of contact surfaces) in contact with a skin surface at given moment, typically just before or during the conducting of the electric current. The device is configured to identify a measure of the quality of the contact between the surfaces of the electrodes and the skin surface at a given moment. The device is configured to adjust the parameters of an electric current through the contact surfaces dependent on the measure of contact quality.

Any suitable method, component or assembly may be used to identify a measure of the total surface area of contact surfaces in contact with the skin surface at a given moment, according to the total number of electrodes present.

In some embodiments, the device is configured to determine the impedance of a circuit comprising the contact electrodes, the ground electrode and a voltage or current supplies a measure of the total surface area of contact surfaces in contact with a skin surface.

In some embodiments, the device is configured to measure the impedance from contact electrodes **10**4, through a skin surface, body and to ground electrode **106.**

The parameters of the following electrical current to provide a desired clinical effect are modified as a function of the thus-measured impedance. In particular, when the measured impedance is indicative of lack of contact with a surface, no current is supplied, even when trigger **118** indicates that a current should be applied. In such embodiments, the impedance measuring functions as a contact detector and switch, allowing supply of electrical current only when there is contact with a skin surface. A device as described herein comprise a single electric circuit comprising all of the contact electrodes, all of the ground electrodes and the power supply. A device as described herein comprise at least two electric circuits, each electric circuit including a power supply, at least one contact electrode and at least one ground electrode. In The parameters of the current supplied by a power supply of a specific electronic circuit when all electrodes of that circuit contact a skin surface (i.e., all of the contact electrodes and also the ground electrodes) are such that a desired level of localized heating is caused.

The shape of the heated zones may be any shape, wherein the width of a zone is defined by the dimensions of contact surface **112,** and the length defined by pulse width and speed of movement of the device over the skin surface. The wound is elliptical.

Device **100** is preferably moved across the skin surface at a speed in the range of from about 0.5 to about 20 cm/sec, more preferably from about 1 to about 10 cm/sec, and most preferably at about 5 cm/sec. According to one non-limiting example, using a contact electrode **102** of power 1 W, with pulse width of about 10 msec and speed of movement of 5cm/sec, a wound of 500 micrometers length is produced per contact electrode and energy delivered to wound area is 10 mJ.

During use of device **100,** ground electrode **106** is in electrical contact with a surface of the person being treated. Ground electrode **106** is of any suitable shape and form. In Fig. 1A, ground electrode **106** is provided in a wrist band **120,** connected to power supply**104** via ground electrode lead **122.** Alternatively, ground electrode **106** may be provided, for example, on an alligator clip, adhesive pad, or the like. As is clear to a person having ordinary skill in the art, the surface area of ground electrode **106** is substantially greater than the sum of contact surfaces **112** of contact electrodes **102,** thereby preventing damage to be caused by the current to a skin surface in contact with ground electrode **106.**

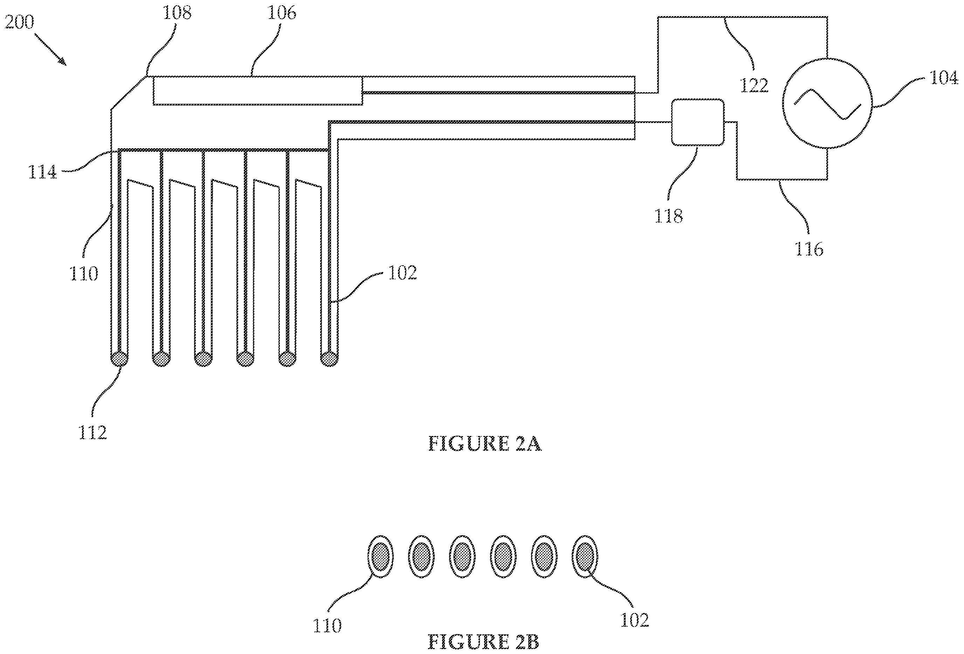

Fig. 2A shows an alternative device. Device **200** is similar to device **100** of Fig. 1A, but with ground electrode **106** provided within housing **108,** and connected to power supply**104** via ground electrode lead **122.** As shown in Fig. 2B, as for Fig. 1B, a contact electrode **102** is embedded in each of teeth **110.** Such embodiments are particularly suitable for self-treatment by a user, wherein ground electrode **106** contacts the hand of the user.

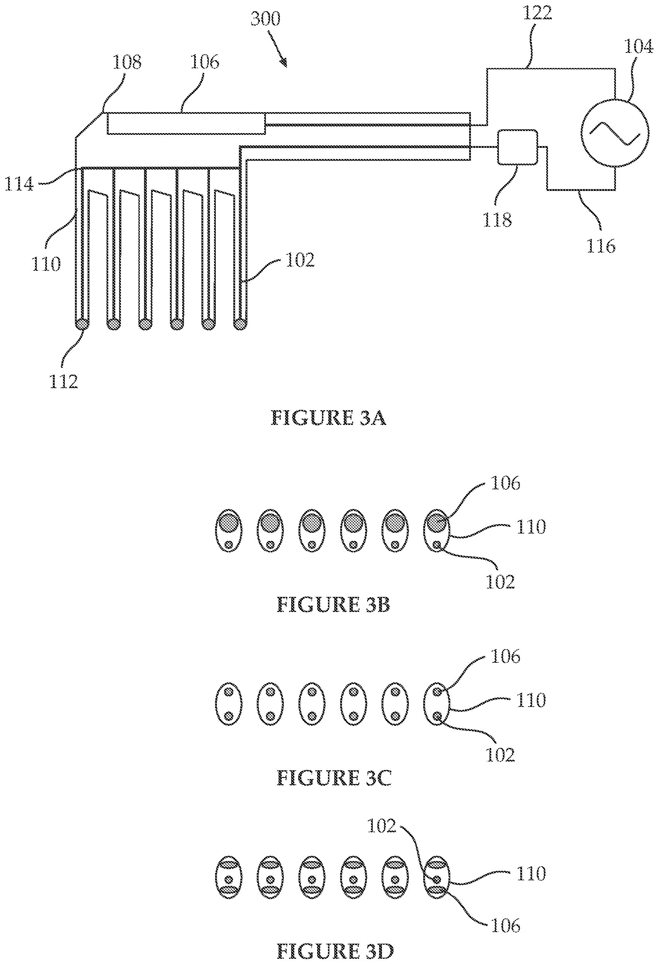

Fig. 3A shows a cross-sectional view of an alternative embodiment **300** of the device of the present invention, wherein a contact electrode **102** and a ground electrode **106,** are both embedded within each of teeth **110.** In some embodiments, as shown in Fig. 3B, the surface area of a ground electrode **106** is substantially larger than that of a contact electrode **102** so that the electrical current causes skin heating only in proximity of a contact electrode **102.** In some embodiments, as shown in Fig. 3C, the surface area of a ground electrode **106** is similar or substantially the same as that of a contact electrode **102** so that the electrical current causes skin heating in proximity of both a contact electrode **102** and a ground electrode **106.** In some embodiments, as shown in Fig. 3D, contact electrode **102,** and at least two ground electrodes **106** are embedded within each of teeth **110.** This configuration provides good contact with contact electrodes **102,** even when the user tilts device **300** forward or backward during use. The electrical current heats the skin either at only contact electrode **102** or also at ground electrodes **106,** depending on the relative surface areas.

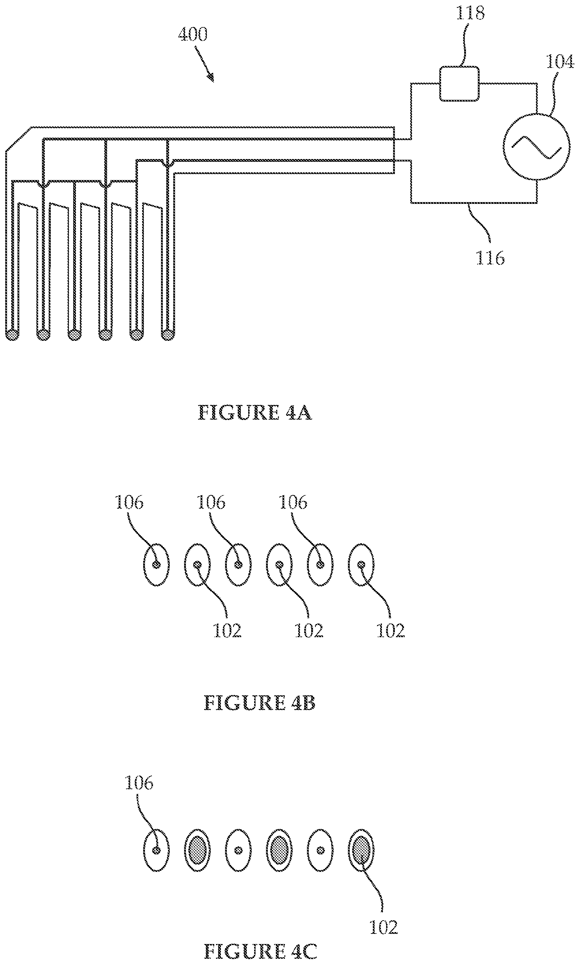

Fig. 4A shows a cross-sectional view of an alternative device 400, wherein adjacent teeth **110** are provided with electrodes of alternating polarity, such that a first of teeth **110** is provided with a contact electrode **102,** a second of teeth **110** is provided with a ground electrode **106,** a third of teeth **110** is provided with a contact electrode **102,** and so on. Analogous to device **300,** the electrical current causes skin heating at only contact electrode **102** or also at ground electrodes **106,** depending on the relative surface areas. The combined surface area of one or more ground electrodes **106** is substantially greater than the combined surface area of contact electrodes **102.** Skin heating is caused only by contact electrodes **102.** Ground electrode **106** is remote from contact electrodes **102.** For example, ground electrode **106** may be located in a wrist band, patch, or the like, or in an extension of housing **108,** such as a handle.

Fig. 4B shows a lower cross-sectional view of the device of Fig. 4A showing a contact electrode embedded in each of the teeth of the device.

Fig. 4C shows a lower cross-sectional view of the device of Fig. 4A showing alternate contact electrodes and ground electrodes embedded in adjacent teeth of the device. The combined surface area of one or more ground electrodes 106 is similar or even equal to the combined surface area of contact electrodes **102.** Wounds are caused by both the contact electrodes **102** and the ground electrodes **106.** Ground electrodes **106** as well as contact electrodes **102** are embedded within teeth **110.** The path of the electrical current is well defined in the vicinity of the contact surfaces. The at least one ground electrode **106** comprises an array of electrodes located proximal to contact electrodes **102.** Any device may be used either in stamping mode (i.e., repeatedly relocated to abutting skin areas and triggered), or in moving mode (i.e. triggered upon travelling a pre-defined distance along the skin surface, or at pre-defined time intervals, substantially as described above). The two-dimensional array of electrodes may comprise two groups comprising electrodes of opposite polarities (i.e. contact electrodes **102** and ground electrodes **106,** respectively). Each of the groups may have equal numbers of electrodes **102** and **106,.** Alternatively, the number of ground electrodes **106** may be greater than the number of contact electrodes.

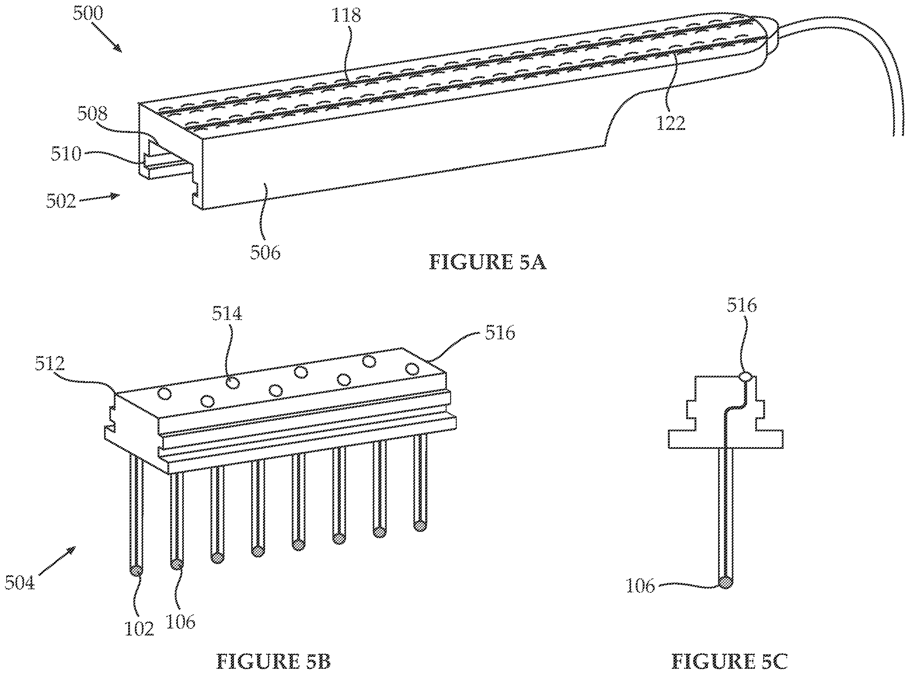

Figs. 5A-5C show an exemplary device **500** comprising an upper first part **502** and a lower second part **504,** parts **502** and **504** being reversibly couplable. First part **502** comprises an elongated member **506** formed from an insulating material, within which are housed contact electrode lead **118** and one or more ground electrode leads **122.** Elongated member **506** is formed with a downwardly-facing longitudinal slot **508** along the length thereof, with two longitudinal grooves **510** on either side, wherein leads **118** and **122** are exposed on the upper surface of slot **508.**

Second part **504** is formed with a rail **512** configured to slidingly engage slot **508** and grooves **510** of upper part **502.** Second part **504** further comprises teeth **110,** downwardly extending from rail **512,** within which are alternatingly embedded contact electrodes **102** and **106.** Conducting contacts **514** and **516** are provided at the upper surface of rail **512,** to provide contact between contact electrodes **102** and contact electrode lead **118,** and ground electrode **106** and ground electrode lead **122,** respectively, when second part **504** is engaged within first part **502.**

Fig. 5C shows a head-on view of second part **504,** showing a ground electrode **106.** As shown, conducting contact **516** for ground electrode **106** is positioned to side of rail **512,** such that when second part **504** is engaged within first part **502,** conducting contact **516** is in contact with ground electrode lead **122.** Conducting contact **514** for contact electrode **102** is positioned at the other side of rail **512** (not shown), such that when second part **504** is engaged within first part **502,** conducting contact **514** is in contact with ground electrode lead **118.** Second part **504,** including electrodes **102** and **106** is disposable. Device **500** comprises a number of interchangeable options for second part **504,** for example, varying in number and/or arrangement and/or inter-electrode distances of contact electrodes **102** and/or ground electrodes **122,** differently-shaped contact surfaces, differently-sized contact surfaces such that one or more users may each select a suitable treatment.

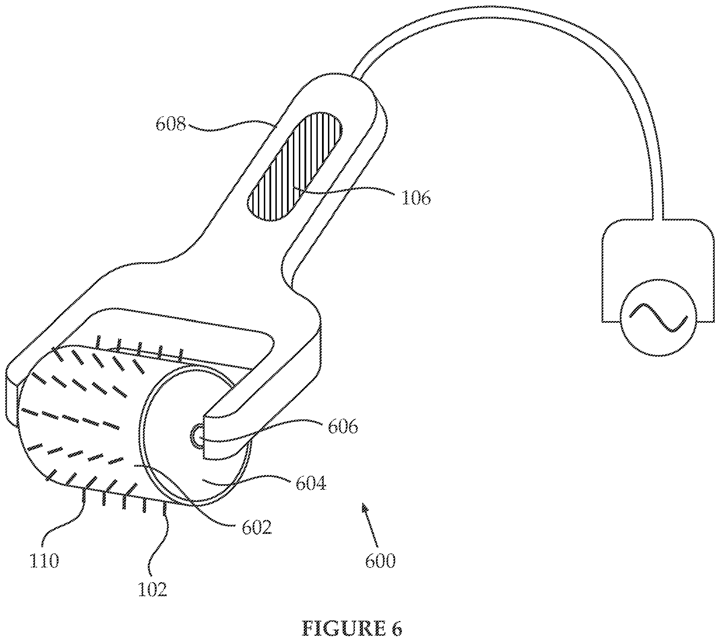

Fig. 6 shows device **600** wherein a plurality of elongated teeth **110** are arranged so as to extend outwards from the outer surface **602** of a cylindrical roller **604.** Each of a plurality of contact electrodes **102,** each having a contact surface **106** are embedded in one of elongated teeth **110** such that contact surface **106** is exposed. Cylindrical roller **602** is arranged around a rotation shaft **606.** A handle **608** constructed from an insulating material extends from both ends of rotation shaft **606,** enabling device **600** to be easily held by a user during operation. Embedded inside handle **608** and exposed for electrical contact with a hand holding handle **608** is ground electrode **106.** Contact electrodes **102** are connected via a power supply **104** to ground electrode **106.** As described above, power supply**104** is configured to supply electric current.

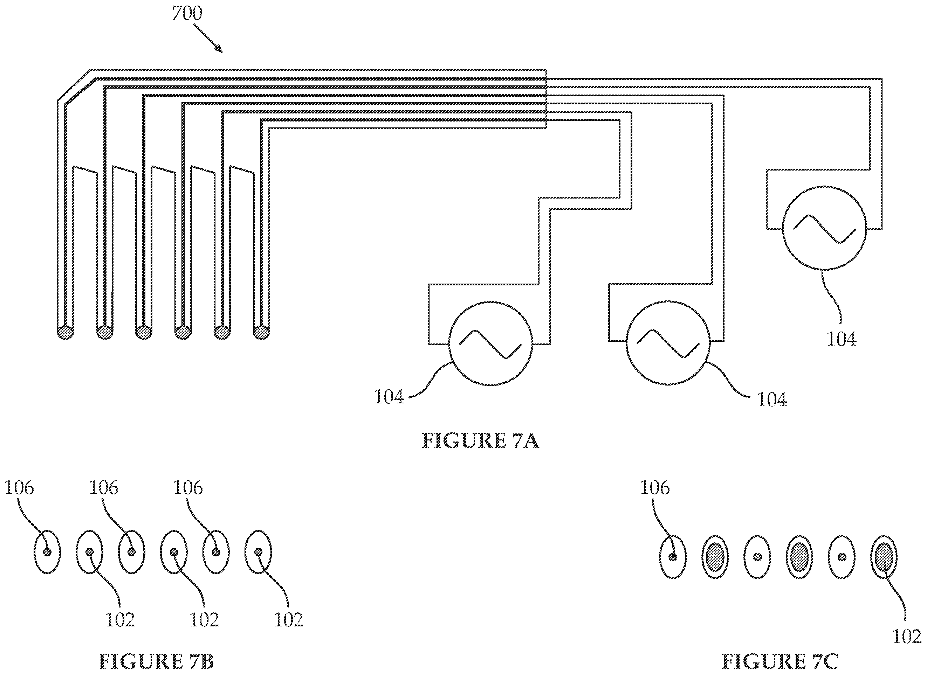

Fig. 7 shows is a schematic representation of operation of device **200** having a power supply **104,** an array of electrodes **1, 2, 3, 4, 5,** and a switching mechanism **700.** Switching mechanism **700** enables the function of each of the five electrodes to independently function as either a contact electrode or a ground electrode. In the example of Fig. 7, switching mechanism **700** is connected in such a way that electrodes **1-4** function as ground electrodes, and electrode 5 as a contact electrode.

### Example

### A pilot study on hair growth stimulation was carried out as follows.

### Method

A test group of eight patients participated in the pilot study: five females aged 39 to 73 with moderate to severe female pattern hair loss, and three males aged 38 to 50 with male pattern hair loss.

All patients (females and males) had previously used Minoxidil® without any success and two of the three male patients had also previously used Finasteride® without any success.

The Ludwig classification of the female patients was II (moderate) - III (extensive). The Norwood classification of the male patients was IV.

Areas of hair loss on the scalp of the subjects were treated using a device substantially similar to that of Figure 4c as described above, at frequency 1MHz every two weeks for a period of at least 8 weeks.. The device was used in stamping mode, with treatment applied to successive 1.5 cm² areas of skin.

The patients were monitored clinically and photographically and were asked to report on any side effects. Dermatoscopic photographs were also taken at each visit.

Patient assessment and satisfaction was rated using the GAIS (Global Aesthetic Improvement Scale) scale after the 4^{th} and the 8^{th} treatment. In this scale, grade 1 indicates exceptional improvement, grade 2 indicates significant improvement, grade 3 indicates moderate improvement, grade 4 indicates no improvement, and grade 5 indicates worsening of the condition.

### Results

Seven of the eight patients have been treated for more than half a year (more than 12 treatments) and one male patient has received five treatments. One patient has been treated for nearly a year

All patients show a remarkable improvement. All patients reported arrest of hair growth, and faster hair growth than before commencement of treatment, as identified by more frequent requirement to visit the hairdresser. All had fuller and more aesthetic hair. In six out of eight patients, many new hair follicles were seen in the treated area after 4-8 treatments.

Patient satisfaction was rated after the 4^{th} and the 8^{th} treatment: Five out of eight patients reported exceptional improvement (GAIS grade 1) and three out of eight reported significant improvement (GAIS grade 2) and were very satisfied with the treatment.

The treatment was reported as being painless, and no side effects were seen. Mild erythema was seen in some patients at the treated area directly after the treatment, which lasted only up to a few hours after the treatment.

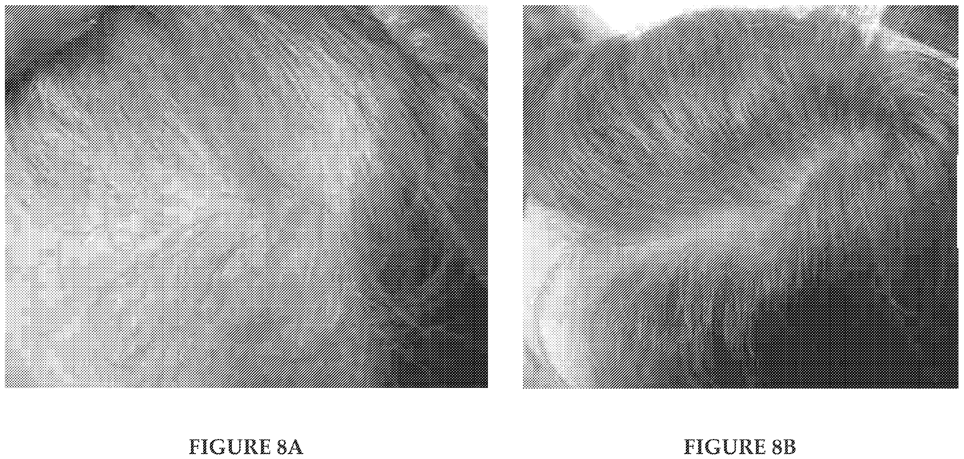

An exemplary photograph of a patient is shown in Figure 8, before treatment (Figure 8A) and after treatment (Figure 8B). In some devices an electrical pulse is triggered every time the device travels a prescribed distance relative to the skin surface. The prescribed distance can be any suitable prescribed distance. The prescribed distance is not more than about 5 mm, not more than about 4 mm and in some embodiments not more than about 3 mm. The prescribed distance is not less than about 0.5 mm and in some embodiments not less than about 0.9 mm. The prescribed distance is between about 1 mm and about 3 mm. When the prescribed distance is smaller, triggerings are closer together while when the prescribed distance is greater, triggerings are further apart. In devices wherein electrical pulse is triggered as a function of distance moved, the method is substantially independent of the speed at which the device is moved. As a result, a user may move the device as convenient, with no need for maintaining a constant speed and may change the speed, for example, to maneuver the device around anatomical features.

It is known in the art to stimulate hair growth by exposing a skin surface to coherent or non-coherent light. In a method as described herein, a skin surface is concurrently or simultaneously irradiated with coherent or non-coherent light to stimulate hair growth together with the teachings described herein. Some devices may further comprise a suitable light source, to implement concurrent or simultaneous irradiation with a coherent or non-coherent light source. Examples of suitable light sources include a laser source, preferably a low level laser light source, for example, a commercially available LED or low power semiconductor laser.

## Claims

1. A device (300) for stimulation of hair re-growth on an area of skin of a subject, the device (300) comprising an array of electrodes
a plurality of protruding elements comprising elongated teeth (110), said array of electrodes comprising
at least one contact electrode (102), and
at least one ground electrode (106),
each of said contact electrodes (102) configured to conduct electrical current to a surface of an area of skin via a contact surface (112) of a said contact electrode (102), wherein said electrical current is adapted to deliver energy sufficient to cause localized heating of
said area of skin,
**characterized in that**
said contact electrode (102) and said ground electrode (106) are both embedded within a distal end of said elongated protruding teeth (110), such that a contact surface (112) of said contact electrode (102) is exposed.

2. The device (300) according to any claim 2, further comprising a power supply (104) configured to supply a voltage causing said electrical current.

3. The device (300) according to claim 2, wherein said power supply (104) is configured to supply said voltage causing said electrical current having a frequency in radio frequency range, optionally wherein said frequency is in the range of from 0.2 MHz to 40 MHz.

4. The device (300) according to claim 3, wherein said power supply (104) is configured so that said electrical current is adapted to carry energy in the range of from 1 to 200 mJ through each said contact surface (112) of each said contact electrode (102).

5. The device (300) according to any of claims 1 to 4, configured such that the function of at least one electrode of said array of electrodes can be switched between being a ground electrode (106) and being a contact electrode (102).

6. The device (300) according to any of claims 1 to 5, wherein said contact surface (112) of each of said contact electrodes (102) has a cross-sectional dimension in the range of from 25 to 500 micrometers.

7. The device (300) according to any of claims 2 to 6, wherein said device (300) is configured to measure the impedance of a circuit comprising said contact electrode (102), said ground electrode (106), said power supply (104) and said skin.

8. The device (300) according to claim 7, wherein said device (300) is configured to adjust parameters of an electric current through said contact surfaces (112) depending on said measured impedance.

9. The device (300) according to claim 1, wherein said protruding elements comprise substantially parallel, elongated elements.

10. The device (300) according to claim 1, wherein a total contact surface area of said contact electrodes (102) is equal to or less than a total surface area of said ground electrodes (106).

11. The device (300) according to any of claims 2 to 10, comprising two parts, a first part comprising a lead connected to said power supply (104) and a second part comprising said contact surfaces (112) of said contact electrodes (102), wherein said first part and said second part are reversibly couplable, wherein in at least one coupled state, there is electrical communication between said lead of said first part and said contact surfaces (112) of said second part, wherein said second part comprising said contact surfaces (112) is disposable.

12. The device (300) according to any of claims 1 to 11, wherein said array of electrodes comprises a two-dimensional array.

## Patentansprüche

1. Vorrichtung (300) zur Stimulation des Nachwachsens von Haar auf einem Hautbereich eines Subjekts, wobei die Vorrichtung (300) eine Anordnung von Elektroden umfasst, eine Vielzahl vorragender Elemente, die längliche Zähne (110) umfassen, wobei die Anordnung von Elektroden
mindestens eine Kontaktelektrode (102) und
mindestens eine Masseelektrode (106) umfasst,
wobei jede der Kontaktelektroden (102) dazu eingerichtet ist, elektrischen Strom über eine Kontaktfläche (112) einer solchen Kontaktelektrode (102) zu einer Oberfläche eines Hautbereichs zu leiten,
wobei der elektrische Strom zur Abgabe von Energie ausgelegt ist, die ausreicht, um ein örtliches Erhitzen des Hautbereichs zu verursachen,
**dadurch gekennzeichnet, dass**
die Kontaktelektrode (102) und die Masseelektrode (106) beide in einem distalen Ende der länglichen vorragenden Zähne (110) eingebettet sind, sodass eine Kontaktfläche (112) der Kontaktelektrode (102) freiliegt.

2. Vorrichtung (300) nach einem der Ansprüche 1 bis 2, weiter eine Energiezufuhr (104) umfassend, die dazu eingerichtet ist, eine Spannung zuzuführen, die den elektrischen Strom verursacht.

3. Vorrichtung (300) nach Anspruch 2, wobei die Energiezufuhr (104) dazu eingerichtet ist, die Spannung zuzuführen, die den elektrischen Strom verursacht, der eine Frequenz im Radiofrequenzbereich aufweist, wobei die Frequenz optional im Bereich von 0,2 MHz bis 40 MHz liegt.

4. Vorrichtung (300) nach Anspruch 3, wobei die Energiezufuhr (104) so eingerichtet ist, dass der elektrische Strom dazu ausgelegt ist, Energie im Bereich von 1 bis 200 mJ durch jede Kontaktfläche (112) jeder Kontaktelektrode (102) zu transportieren.

5. Vorrichtung (300) nach einem der Ansprüche 1 bis 4, so eingerichtet, dass die Funktion von mindestens einer Elektrode der Anordnung von Elektroden zwischen einer Anwendung als Masseelektrode (106) und einer Anwendung als Kontaktelektrode (102) umgeschaltet werden kann.

6. Vorrichtung (300) nach einem der Ansprüche 1 bis 5, wobei die Kontaktfläche (112) jeder der Kontaktelektroden (102) eine Querschnittsabmessung im Bereich von 25 bis 500 Mikrometern aufweist.

7. Vorrichtung (300) nach einem der Ansprüche 2 bis 6, wobei die Vorrichtung (300) dazu eingerichtet ist, die Impedanz eines Schaltkreises zu messen, der die Kontaktelektrode (102), die Masseelektrode (106), die Energiezufuhr (104) und die Haut umfasst.

8. Vorrichtung (300) nach Anspruch 7, wobei die Vorrichtung (300) dazu eingerichtet ist, Parameter eines elektrischen Stroms durch die Kontaktflächen (112) abhängig von der gemessenen Impedanz einzustellen.

9. Vorrichtung (300) nach Anspruch 1, wobei die vorragenden Elemente im Wesentlichen parallele, längliche Elemente umfassen.

10. Vorrichtung (300) nach Anspruch 1, wobei eine Gesamtkontaktfläche der Kontaktelektroden (102) gleich oder kleiner als eine Gesamtfläche der Masseelektroden (106) ist.

11. Vorrichtung (300) nach einem der Ansprüche 2 bis 10, umfassend zwei Teile, einen ersten Teil, der eine mit der Energiezufuhr (104) verbundene Leitung umfasst, und einen zweiten Teil, der die Kontaktflächen (112) der Kontaktelektroden (102) umfasst, wobei der erste Teil und der zweite Teil reversibel koppelbar sind, wobei in mindestens einem gekoppelten Zustand eine elektrische Kommunikation zwischen der Leitung des ersten Teils und den Kontaktflächen (112) des zweiten Teils vorliegt, wobei der die Kontaktflächen (112) umfassende zweite Teil frei verfügbar ist.

12. Vorrichtung (300) nach einem der Ansprüche 1 bis 11, wobei die Anordnung von Elektroden eine zweidimensionale Anordnung umfasst.

## Revendications

1. Dispositif (300) pour la stimulation d'une repousse des cheveux sur une zone de la peau d'un sujet, le dispositif (300) comprenant :
un réseau d'électrodes ;
plusieurs éléments faisant saillie comprenant des dents allongées (110), ledit réseau d'électrodes comprenant :
au moins une électrode de contact (102) ; et
au moins une électrode de masse (106) ;
chacune desdites électrodes de contact (102) étant configurée pour conduire un courant électrique jusqu'à une surface d'une zone de la peau à travers une surface de contact (112) d'une desdites électrodes de contact (102) ;
dans lequel ledit courant électrique est conçu pour distribuer une énergie suffisante pour obtenir un chauffage localisé de ladite zone de la peau ;
**caractérisé en ce que**
ladite électrode de contact (102) et ladite électrode de masse (106) sont toutes deux incorporées dans une extrémité distale desdites dents allongées (110) faisant saillie, d'une manière telle qu'une surface de contact (112) de ladite électrode de contact (102) est exposée.

2. Dispositif (300) selon l'une quelconque des revendications 1 à 2, comprenant en outre une alimentation électrique (104) configurée pour alimenter une tension générant ledit courant électrique.

3. Dispositif (300) selon la revendication 2, dans lequel ladite alimentation électrique (104) est configurée pour alimenter ladite tension faisant en sorte que ledit courant électrique possède une fréquence dans la plage des fréquences radio, de manière facultative dans lequel ladite fréquence se situe dans la plage de 0,2 MHz à 40 MHz.

4. Dispositif (300) selon la revendication 3, dans lequel ladite alimentation électrique (104) est configurée d'une manière telle que ledit courant électrique est conçu pour transporter de l'énergie dans la plage de 1 à 200 mJ à travers chacune desdites surfaces de contact (112) de chacune desdites électrodes de contact (102).

5. Dispositif (300) selon l'une quelconque des revendications 1 à 4, configuré d'une manière telle que la fonction d'au moins une électrode parmi ledit réseau d'électrodes peut faire l'objet d'une commutation entre le fait de représenter une électrode de masse (106) et le fait de représenter une électrode de contact (102).

6. Dispositif (300) selon l'une quelconque des revendications 1 à 5, dans lequel ladite surface de contact (112) de chacune desdites électrodes de contact (102) possède une dimension en section transversale qui se situe dans la plage de 25 à 500 µm.

7. Dispositif (300) selon l'une quelconque des revendications 2 à 6, dans lequel ledit dispositif (300) est configuré pour mesurer l'impédance d'un circuit comprenant ladite électrode de contact (102), ladite électrode de masse (106), ladite alimentation électrique (104) et ladite peau.

8. Dispositif (300) selon la revendication 7, dans lequel ledit dispositif (300) est configuré pour régler des paramètres d'un courant électrique à travers lesdites surfaces de contact (112) en fonction de ladite impédance mesurée.

9. Dispositif (300) selon la revendication 1, dans lequel lesdits éléments faisant saillie comprennent des éléments allongés essentiellement parallèles.

10. Dispositif (300) selon la revendication 1, dans lequel une aire de surface de contact totale desdites électrodes de contact (102) est égale ou inférieure à une aire de surface totale de ladite électrode de masse (106).

11. Dispositif (300) selon l'une quelconque des revendications 2 à 10, comprenant deux parties, une première partie comprenant un conducteur relié à ladite alimentation électrique (104) et une seconde partie comprenant lesdites surfaces de contact (112) desdites électrodes de contact (102), ladite première partie et ladite seconde partie pouvant être couplées de manière réversible, dans lequel, dans au moins un état couplé, on obtient une communication électrique entre ledit conducteur de ladite première partie et lesdites surfaces de contact (112) de ladite seconde partie, ladite seconde partie comprenant lesdites surfaces de contact (112) étant jetable.

12. Dispositif (300) selon l'une quelconque des revendications 1 à 11, dans lequel ledit réseau d'électrodes comprend un réseau en deux dimensions.
